# EUROPEAN PATENT APPLICATION

(11) **EP 3 954 329 A1**
(43) Date of publication of application: **16.02.2022**
(21) Application number: 20787097.3
(22) Date of filing: 03.04.2020
(51) Int. Cl.: A61F 2/24

(54) **STENT USED FOR IMPLANTING VALVE-IN-VALVE**

(30) Priority: 08.04.2019 CN 201910274453
(71) Applicant: Beijing Balance Medical Technology Co., Ltd., Beijing 102200 (CN)
(72) Inventor: JIN, Lei, Beijing 102200 (CN); FAN, Zhihao, Beijing 102200 (CN); MU, Hong, Beijing 102200 (CN)
(74) Representative: Murgitroyd & Company
(86) International application number: PCT/CN2020/083086
(87) International publication number: WO 2020/207330

(57) **Abstract**

The present invention relates to a stent for interventional valve-in-valve, wherein the stent is a metal mesh tube, and is provided with four rows of transversely extending circumferential struts and a plurality of columns of axial struts arranged between the circumferential struts; the axial struts in each row are arranged in a staggered mode, the axial struts are connected with transverse struts attached thereon to form a staggered honeycomb meshes, the area of honeycomb meshes at the inflow end is basically the same as that of the honeycomb meshes in the middle row, and the area of honeycomb meshes at the outflow end is slightly larger than that of the honeycomb meshes in the other three rows. According to a stent for an interventional valve-in-valve provided herein, in view of the specialty that interventional valve-in-valves are implanted into the previously implanted damaged surgical valve or interventional valve by intervention and in close attachment with the failed valve, the subversive improvement is carried out on the conventional interventional valve stent, with all meshes of the stent adopting honeycomb-like structures, so that the stent with the structure can realize certain rigidity, has high synchronous deployment speed, good attachment, and better use effect.

## Description

### Technical Field

The invention relates to the technical field of medical instruments, in particular to a connection structure between a stent and a valve leaflet for an interventional valve-in-valve or an interventional pulmonary valve, and an interventional valve-in-valve and an interventional pulmonary valve applying the connecting structure.

### Background Art

With the increasing economic level in China, the replacement of bioprosthetic valves in elderly patients with the valvular disease has increased year by year, and the proportion of bioprosthetic valve application continues to approach developed countries. 2017 Guidelines released by AHC/ACC reduce the age of patients surgically implanted with a bioprosthetic valve to 50 years of age, and for patients of any age who have contraindications to anticoagulant therapy, are unsuitable for anticoagulant therapy or are unwilling to receive anticoagulant therapy, the use of biological valves is recommended. In addition, the application of transcatheter interventional bioprosthetic valves in China has been increasing year by year in recent years. Since various implanted (interventional) bioprosthetic valves are still facing uncertainties in durability, some patients will inevitably experience damage or calcification of the bioprosthetic valve after surgery, resulting in loss of function. To this end, the interventional valve-in-valve will provide re-interventional therapy for such patients.

Interventional bioprosthetic heart valves are placed into various kinds of previously implanted damaged interventional bioprosthetic valves through the femoral artery or thoracotomy minimally invasive intervention, so as to replace the original valve function, which has been successfully applied in the clinic. With the addition of these clinical cases, perivalvular leakage between the re-inserted bioprosthetic valve and the original failed valve was found to be one of the common complications. In this regard, it is desirable to implant a re-interventional valve-in-valve after the failure of the interventional artificial bioprosthetic valve. The present invention aims to provide a stent for developing an interventional valve-in-valve.

### Summary of the Invention

In view of this, the technical problem to be solved by the present invention is to provide a stent for interventional valve-in-valve, which has three or four rows of honeycomb meshes and can achieve fast and synchronous opening speed.

In order to solve the above technical problems, a first technical solution adopted by the present invention is as follows: a stent for interventional valve-in-valve is a metal mesh tube and is provided with four rows of transversely extending circumferential struts and a plurality of columns of axial struts arranged between the circumferential struts; the axial struts in each row are arranged in a staggered mode, wherein the first and second rows of circumferential struts on the lower side define an inflow end of the stent, and the third and fourth rows of circumferential struts define an outflow end of the stent; the circumferential struts in each row are formed by connecting a plurality of groups of angled struts, and each group of struts is in a deformable V-shape, with a deformation angle of 0-90 degrees; the axial struts are connected with transverse struts attached thereon to form a staggered honeycomb meshes, an area of the honeycomb meshes at the inflow end is basically the same as that of the honeycomb meshes in the middle row, and an area of honeycomb meshes at the outflow end is slightly larger than that of the honeycomb meshes in the other two rows.

Furthermore, the area of honeycomb meshes at the outflow end is 10%-20% larger than that of the honeycomb meshes in the other two rows.

Furthermore, the area of honeycomb meshes at the inflow end differs by no more than 10% from that in the middle row.

Furthermore, the stent has a height of 13-25 mm, an inner diameter of 18-30 mm, and a wall thickness of 0.35-0.65 mm.

Furthermore, the plurality of groups of axial struts have the same size, the axial struts of honeycomb meshes at the inflow end and in the middle row are close in size, and the axial struts of honeycomb meshes at the outflow end are slightly larger than that of the honeycomb meshes in the other two rows.

A second technical solution adopted by the present invention is as follows: a stent for interventional valve-in-valve is a metal mesh tube and is provided with five rows of transversely extending circumferential struts and a plurality of columns of axial struts arranged between the circumferential struts; the axial struts in each row are arranged in a staggered mode, wherein the first and second rows of circumferential struts on the lower side define an inflow end of the stent, and the fourth and fifth rows of circumferential struts define an outflow end of the stent; the circumferential struts in each row are formed by connecting a plurality of groups of angled struts, and each group of struts is in a deformable V-shape, with a deformation angle of 0-90 degrees; the axial struts are connected with transverse struts attached thereon to form a staggered honeycomb meshes, an area of the honeycomb meshes at the inflow end is basically the same as that of the honeycomb meshes in the middle row, and an area of honeycomb meshes at the outflow end is slightly larger than that of the honeycomb meshes in the other three rows.

Furthermore, the area of honeycomb meshes at the outflow end is 10%-20% larger than that of the honeycomb meshes in the other three rows.

Furthermore, the area of honeycomb meshes at the inflow end differs by no more than 10% from that in the middle row.

Further, the stent has a height of 13-25 mm, an inner diameter of 18-30 mm, and a wall thickness of 0.35-0.65 mm.

Furthermore, the plurality of groups of axial struts are the same size, the axial struts at the inflow end and in the middle row of honeycomb meshes are close in size, and the axial struts at the outflow end and the honeycomb meshes are slightly larger than that of honeycomb meshes in the other three rows.

The invention has the beneficial effects that: 1. the interventional valve-in-valve has better rapid uniform unfolding characteristics, and can be easily and accurately anchored in a previously failed bioprosthetic valve; 2. compared with the interventional aortic valve in the failed bioprosthetic valve, the symmetry of the structure endows the interventional valve-in-valve better adherence, realizing the close attachment with the original failed valve to avoid paravalvular leakage; and 3. the structure is designed as a connection between the valve leaflets to make the fixing of the valve leaflets on the stent more reasonable and firmer, achieving the same durability as the surgical bioprosthetic valve in the enterprise.

### Brief Description of the Drawings

FIG. 1 is a schematic structural diagram of the interventional valve-in-valve in the prior art.
FIG. 2 is a schematic structural diagram of a stent for interventional valve-in-valve according to an example of the present invention;
FIG. 3 is an expanded plan view of a stent for interventional valve-in-valve according to an embodiment of the present invention.

### Detailed Description of the Invention

The specific embodiments are described below with specific embodiments. It should be understood that the specific examples described herein are merely illustrative of the present invention and are not intended to limit the present invention.

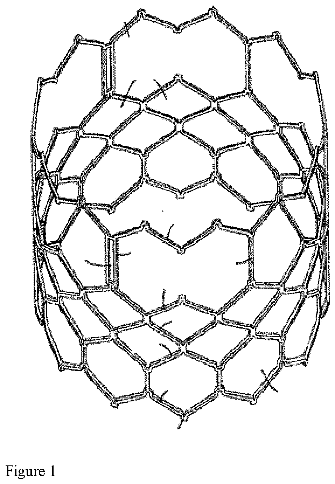

Interventional valve-in-valve is commonly used in previously surgically implanted or intervened failed bioprosthetic heart valves (including four valve-implanted or intervened bioprosthetic valves) to achieve re-interventional treatment of the heart valve. The structure of the existing stent for interventional valve-in-valve is shown in FIG. 1.

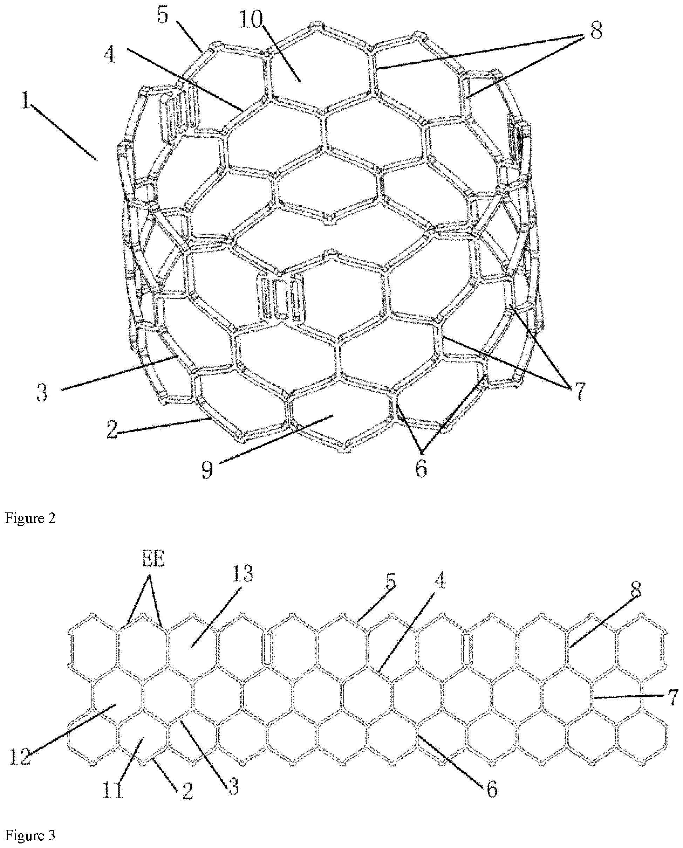

Referring to FIGs. 2 and 3, a stent 1 for interventional valve-in-valve is a metal mesh tube and is provided with four rows of transversely extending circumferential struts 2, 3, 4, 5, and a plurality of axial struts 6, 7, 8 arranged between each row of circumferential struts; the axial struts in each row are arranged in a staggered mode, wherein the first and second rows of circumferential struts 2, 3 on the lower side define an inflow end 9 of the stent, and the third and fourth rows of circumferential struts 4, 5 define an outflow end 10 of the stent; the circumferential struts in each row are formed by connecting a plurality of groups of angled struts EE, and each group of struts EE is in a deformable V-shape, with a deformation angle of 0-90 degrees; the axial struts are connected with transverse struts attached thereon to form a staggered honeycomb-like meshes, the area of honeycomb meshes 11 at the inflow end and the honeycomb meshes 12 in the middle row are the same or slightly different, with the area difference between the two by no more than 10%, and the area of honeycomb meshes at the outflow end 13 is slightly larger than that of the honeycomb meshes in the other two rows, by 10%-20%. Generally, different areas of the honeycomb meshes are achieved by different heights of the axial struts.

In some cases, where a better interventional valve-in-valve is desired, the stent may be augmented with a row of honeycomb mesh. Generally, the stent has a height of 13-25 mm, an inner diameter of 18-30 mm, and a wall thickness of 0.35-0.65 mm.

Other structures of the interventional valve-in-valve in the present embodiment, such as a connecting post structure connected to the valve leaflet, may employ the same or similar structures as in the prior art.

Finally, it should be noted that the foregoing examples are merely intended for describing the technical solutions of the present invention, but not for limiting the present invention. Although the present invention is described in detail with reference to the foregoing examples, persons of ordinary skill in the art should understand that they may still make modifications to the technical solutions described in the foregoing examples or make equivalent replacements to some or all technical features thereof, without departing from the scope of the technical solutions of the examples of the present invention.

## Claims

1. A stent for interventional valve-in-valve, **characterized in that** the stent is a metal mesh tube and is provided with four rows of transversely extending circumferential struts and a plurality of columns of axial struts arranged between the circumferential struts; the axial struts in each row are arranged in a staggered mode, wherein the first and second rows of circumferential struts on the lower side define an inflow end of the stent, and the third and fourth rows of circumferential struts define an outflow end of the stent; the circumferential struts in each row are formed by connecting a plurality of groups of angled struts, and each group of struts is in a deformable V-shape, with a deformation angle of 0-90 degrees; the axial struts are connected with transverse struts attached thereon to form a staggered honeycomb meshes, an area of the honeycomb meshes at the inflow end is basically the same as that of the honeycomb meshes in the middle row, and an area of honeycomb meshes at the outflow end is slightly larger than that of the honeycomb meshes in the other two rows.

2. The stent for interventional valve-in-valve according to claim 1, **characterized in that** the area of honeycomb meshes at the outflow end is larger than that of the honeycomb meshes in the other two rows by 10%-20%.

3. The stent for interventional valve-in-valve according to claim 1, **characterized in that** the area of honeycomb meshes at the inflow end differs by no more than 10% from that of the honeycomb mesh in the middle row.

4. The stent for interventional valve-in-valve according to claim 1, **characterized in that** the stent has a height of 13-25 mm, an inner diameter of 18-30 mm, and a wall thickness of 0.35-0.65 mm.

5. The stent for interventional valve-in-valve according to claim 1, **characterized in that** the plurality of groups of axial struts have the same size, the axial struts of honeycomb meshes at the inflow end and in the middle row are close in size, and the axial struts of honeycomb meshes at the outflow end are slightly larger in size than that of the honeycomb meshes in the other two rows.

6. A stent for interventional valve-in-valve, **characterized in that** the stent is a metal mesh tube and is provided with five rows of transversely extending circumferential struts and a plurality of columns of axial struts arranged between the circumferential struts; the axial struts in each row are arranged in a staggered mode, wherein the first and second rows of circumferential struts on the lower side define an inflow end of the stent, and the fourth and fifth rows of circumferential struts define an outflow end of the stent; the circumferential struts in each row are formed by connecting a plurality of groups of angled struts, and each group of struts is in a deformable V-shape, with a deformation angle of 0-90 degrees; the axial struts are connected with transverse struts attached thereon to form a staggered honeycomb meshes, the area of honeycomb meshes at the inflow end is basically the same as that of the honeycomb meshes in the middle row, and the area of honeycomb meshes at the outflow end is slightly larger than that of the honeycomb meshes in the other three rows.

7. The stent for interventional valve-in-valve according to claim 6, **characterized in that** the area of honeycomb meshes at the outflow end is larger than that of the honeycomb meshes in the other three rows by 10%-20%.

8. The stent for interventional valve-in-valve according to claim 6, **characterized in that** the area of honeycomb meshes at the inflow end differs by no more than 10% from that of the honeycomb meshes in the middle row.

9. The stent for interventional valve-in-valve according to claim 6, **characterized in that** the stent has a height of 13-25 mm, an inner diameter of 18-30 mm, and a wall thickness of 0.35-0.65 mm.

10. The stent for interventional valve-in-valve according to claim 6, **characterized in that** the plurality of groups of axial struts have the same size, the axial struts of honeycomb meshes at the inflow end and in the middle row are close in size, and the axial struts of honeycomb meshes at the outflow end are slightly larger than that of the honeycomb meshes in the other three rows.
